# FASCICULE DE BREVET EUROPEEN

(11) **EP 1 797 124 B1**
(45) Date de publication et mention de la délivrance du brevet: **01.09.2010**
(21) Numéro de dépôt: 05792279.1
(22) Date de dépôt: 03.10.2005
(51) Int. Cl.: C07K 14/78, A61L 27/24, A61L 24/00, A61L 17/08

(54) **GREFFAGE COVALENT DE SUBSTANCES HYDROPHOBES SUR LE COLLAGENE**
KOVALENTES GRAFTING VON HYDROPHOBEN SUBSTANZEN AUF COLLAGEN
COVALENT GRAFTING OF HYDROPHOBIC SUBSTANCES ON COLLAGEN

(30) Priorité: 04.10.2004 US 615783 P; 05.11.2004 FR 0411793
(43) Date de publication de la demande: 20.06.2007
(73) Titulaire: BIOM'UP, 69800 Saint Priest (FR)
(72) Inventeur: GAGNIEU, Christian, F-69680 Chassieu (FR)
(74) Mandataire: Tetaz, Franck Claude Edouard
(86) Numéro de dépôt international: PCT/EP2005/054972
(87) Numéro de publication internationale: WO 2006/037770

(56) Documents cités:
- EP-A- 0 212 209
- KAMYSHNY A ET AL: "Adsorption of hydrophobized IgG and gelatin onto phosphatidyl choline-coated silica" COLLOIDS AND SURFACES B: BIOINTERFACES 1999 NETHERLANDS, vol. 13, no. 4, 1999, pages 187-194, XP009060041 ISSN: 0927-7765
- TOLEDANO O ET AL: "Emulsification and foaming properties of hydrophobically modified gelatin" JOURNAL OF COLLOID AND INTERFACE SCIENCE 15 APR 1998 UNITED STATES, vol. 200, no. 2, 15 avril 1998 (1998-04-15), pages 235-240, XP002363169 ISSN: 0021-9797
- TOLEDANO O ET AL: "Formation of surface active gelatin by covalent attachment of hydrophobic chains" JOURNAL OF COLLOID AND INTERFACE SCIENCE 1997 UNITED STATES, vol. 193, no. 2, 1997, pages 172-177, XP002363170 ISSN: 0021-9797
- MAGDASSI ET AL.: "Interfacial properties of hydrphobically modified biomolecules: fundamental aspects and applications" JOURNAL OF DISPERSION SCIENCE AND TECHNOLOGY, vol. 22, no. 4, 2001, pages 313-322, XP009060047
- CHOGLAY ASHRAF A ET AL: "Procollagen binding to sphingomyelin" JOURNAL OF BIOLOGICAL CHEMISTRY, vol. 268, no. 9, 1993, pages 6107-6114, XP002331086 ISSN: 0021-9258
- ROUSSEAU C F ET AL: "In vitro cytocompatibility of porcine type I atelocollagen crosslinked by oxidized glycogen" BIOMATERIALS, ELSEVIER SCIENCE PUBLISHERS BV., BARKING, GB, vol. 23, no. 6, 15 mars 2002 (2002-03-15), pages 1503-1510, XP004348160 ISSN: 0142-9612
- KARUBE I ET AL: "EFFECT OF ALCOHOLS ON THE COLLAGEN PHOSPHATIDYL CHOLINE INTERACTION" BIOCHIMICA ET BIOPHYSICA ACTA, vol. 581, no. 1, 1979, pages 106-113, XP002331087 ISSN: 0006-3002

## Description

La présente invention concerne un collagène greffé hydrophobe, son procédé de préparation et son utilisation, notamment en thérapie. Dans la présente invention, des substances ou molécules à caractère hydrophobe sont greffées par des liaisons covalentes sur des résidus acides-aminés réactifs de molécules collagéniques. Les couplages chimiques ont pour but de modifier les propriétés physico-chimiques et biologiques du collagène et/ou de ses dérivés. En particulier, l'introduction de résidus hydrophobes permet de moduler le caractère hydrophile du collagène et de modifier ses propriétés chimiotactiques impliquées dans l'adhésion et la croissance cellulaires.

Peu ou pas de greffage sur le collagène sont connus de l'état de la technique en dehors des greffages de peptide d'adhésion sur le collagène avec augmentation de l'adhésion cellulaire. Le greffage étant réalisé par un procédé chimique particulier [1]. Certains auteurs décrivent aussi des greffages de collagène sur des substances inertes tel que le polyuréthane par des procédés très spécifique au produit et à l'application [2]. Par ailleurs des mélanges de collagène et d'acides gras existent mais aucun greffage d'acides gras sur du collagène, en particulier par liaison covalente n'est rapporté dans la littérature.

Les molécules de collagène sont des protéines animales situées dans la matrice extracellulaire qui possèdent dans leur structure un ou plusieurs domaines en triple hélice. La triple hélice est obtenue par association de trois chaînes alpha composées chacune de 1050 acides aminés. A l'extrémité des chaînes, des zones non hélicoïdales d'une quarantaine d'acides aminés permettent l'association des fibres de collagène entre elles. Ce sont les télopeptides. Ces protéines sont caractérisées par leur richesse en glycine (33%) et par la présence pour environ 30% de proline et d'hydroxyproline.

Pour la réalisation et la confection de biomatériaux, les collagènes de plusieurs types et de différents niveaux de structuration sont extraits des tissus sources par des procédés bien connus.
↓ Le collagène est dit natif lorsque l'ensemble de la structure qu'il adopte dans les tissus (triple hélice et télopeptides) est conservé à l'extraction.
↓ Le collagène peut être clivé de façon enzymatique ou chimique au niveau des télopeptides : le collagène est alors appelé atelocollagène.
↓ Lorsque les trois chaînes alpha de la triple hélice sont séparé es par dénaturation (chauffage par exemple), le collagène est dit dénaturé.
↓ Quant à la gélatine, elle est caractérisée par une dénaturation du collagène et une hydrolyse (chimique ou thermique) des chaînes alpha en fragments peptidiques.

Différents types de collagène ont été mis en évidence et certains ont été isolés et produits industriellement (essentiellement le type I et le type IV).

Le collagène présente des propriétés physico-chimiques et biologiques variées qui font de lui une matière première de choix pour la confection de biomatériaux. Ainsi, il présente des propriétés rhéologiques spécifiques, une faible antigénicité, un rôle dans la croissance et la différenciation cellulaires, et aussi un fort pouvoir hémostatique. Dans les différents domaines de la médecine et plus particulièrement de la chirurgie, les biomatériaux sont très couramment utilisés. En général, l'adhésion et l'intégration cellulaire sont recherchées. Pourtant, depuis quelques années, l'accent a été mis sur la mise au point de matériaux réduisant l'adhésion cellulaire. Des phénomènes d'adhérences post-chirurgicales à des biomatériaux peuvent apparaître en plus des adhérences conséquences intrinsèques de la chirurgie. De nombreuses études sont en cours pour mettre au point des systèmes permettant de réduire ou d'éliminer les phénomènes d'adhérences.

Selon l'état de la technique, la diminution de l'adhésion cellulaire sur les biomatériaux peut être obtenue par modification de leurs propriétés de surface. La charge de la surface, la rugosité, l'exposition de certaines structures chimiques et l'hydrophobicité sont des facteurs clés de la régulation de l'adhésion cellulaire. En effet, des surfaces chargées négativement induisent une répulsion des cellules chargées elles aussi négativement [3, 4] et entraînent une réduction de l'adhésion cellulaire. La rugosité du substrat joue aussi un rôle clé puisque des surfaces lisses sont anti-adhérentes [5, 6]. Le contrôle de l'adhésion cellulaire peut aussi être obtenu en greffant des structures chimiques ou biochimiques qui ont une influence directe sur les événements moléculaires qui ont lieu lors de l'interaction des cellules avec les matériaux.

Ainsi, l'adhésion cellulaire peut être augmenté par greffage sur des surfaces inertes des matériaux connus pour leurs propriétés favorisant l'adhésion comme du collagène [7], de l'hydroxyapatite [5], des polylysines [4], des polymères hydroxylés ou de peptides de surface adhérents [8].

De manière similaire, trois stratégies majeures sont mises en place pour diminuer l'adhésion cellulaire :
- le greffage sur des polymères inertes de molécules bioactives avec un pouvoir anti-adhérent comme l'héparine ou la thapsigargine qui affectent directement la réorganisation cellulaire requise pour l'adhésion cellulaire [9, 10],
- le greffage de substances hydrophobes comme le Téflon [11], la polyvinylpyrrolidone ou le polyacrylamide [12] sur du PMMA le plus généralement,
- la modification de l'hydrophobie de surface par n'importe quel moyen puisqu'il a été montré que des polymères synthétiques de plus en plus hydrophobes induisent une diminution de l'adhésion cellulaire [3, 6, 13, 14].

Des gélatines greffées par des acides gras allant jusqu'à 16 atomes de carbone sont décrites par Toledano & al. (J. of Colloid and Interface Science, 193, 172-177 (1997) et 200, 235-240 (1998)), par Kamyshny & al. (Colloids and Surfaces B: Biointerfaces, 13 (1999) 187-194) et Magdassi & al. (J. Dispersion Science and Technology, 22(4), 313-322 (2001) pour l'étude de leurs propriétés interfaciales.

Bien que le collagène soit connu et employé pour ses propriétés favorisant l'adhésion, l'objet de la présente invention consiste en un nouveau produit présentant des propriétés antiadhésives, comprenant des collagènes hydrophobes tout aussi biocompatibles que le collagène de départ et présentant l'essentiel des autres propriétés biologiques et rhéologiques du collagène excepté son action sur l'adhésion et la croissance cellulaires. Les substances hydrophobes greffées sont préférentiellement des acides gras saturés ou non. En fonction du choix des acides gras employés pour ce greffage, le collagène greffé selon l'invention est dégradé en substances parfaitement reconnues par l'organisme humain sans aucune réaction pathologique.

La présente invention concerne donc un collagène hydrophobe greffé comprenant des acides gras greffés sur le collagène par liaison covalente, pour son utilisation en thérapie. De manière préférentielle, les acides gras sont greffés sur les résidus amines libres de la chaîne alpha du collagène, en particulier sur les amines libres des résidus lysyles de la chaîne alpha du collagène.

Le pourcentage d'acides gras par rapport aux résidus amines libres de la chaîne alpha du collagène est compris entre 1 et 100%, de préférence supérieur à environ 10% et inférieur à environ 85%. Selon un mode plus préférentiel de réalisation de l'invention, le pourcentage d'acides gras est compris entre 15 et 50%, plus préférentiellement compris entre 20 et 30%.

Le collagène greffé selon l'invention est un collagène de toute origine, notamment un collagène natif, atelocollagène natif ou dénaturé ou de la gélatine. De manière avantageuse, le collagène greffé est un collagène d'origine mammifère de préférence d'origine porcine, qui aura subi avantageusement un traitement prophylactique approprié pour détruire les agents pathogènes.

Le présent document concerne également un procédé de préparation d'un collagène hydrophobe greffé tel que défini ci-dessus et ci-après, dans lequel on fait réagir une quantité appropriée d'un acide gras activé avec le collagène dans un milieu réactionnel approprié.

Dans le procédé de greffage, l'activation de la fonction carboxylique de l'acide gras est préférentiellement obtenue par la formation d'une liaison ester activée ou d'un imidazolide. L'acide gras ainsi activé réagit avec les amines déprotonées des résidus epsilon lysines des chaînes alpha du collagène. L'acide gras activé peut être soit cristallisé soit préparé extemporanément en solution.

L'acide gras activé peut être obtenu par réaction stoechiométrique du carbonyldiimidazole (CDI) sur l'acide gras dans le diméthylformamide (DMF) ou le dimethylsulfoxide (DMSO). Lorsque la réaction d'activation est réalisée dans le DMF, le produit activé est cristallisé et isolé et ajouté sous forme solide à la solution de collagène à greffer. Lorsque l'acide gras activé est préparé dans le DMSO, il est ajouté en solution au collagène. La préparation de l'acide gras activé dans le DMF peut être utilisée pour synthétiser l'ensemble des acides gras compris entre C12 et C22. Pour tous les autres mais aussi pour ceux là également, l'activation est possible dans le DMSO. Le rendement de la réaction d'activation est supérieur à 95% et l'acide gras activé ne présente pas de perte d'activité mesurable après 18 mois de stockage à 4°C. La formule chimique de l'acide gras activé (imidazolide) peut être représentée par la formule I ci-après : 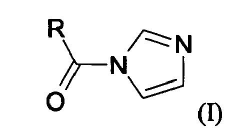 dans laquelle R représente la chaîne hydrocarbonée de l'acide gras.

L'activation de l'acide gras peut également être réalisée par réaction du N-hydroxysuccinimide sur l'acide gras précédé d'une activation avec un carbodiimide tel que le dicyclohexylcarbodiimide ou le diisopropylcarbodiimide. L'acide gras activé ainsi isolé peut être greffé de la même façon que précédemment sur le collagène. La formule chimique de l'acide gras (succinimidyl) activé peut être représentée par la formule II ci-après : 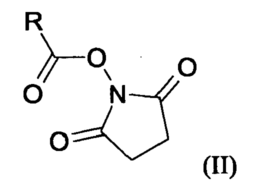 dans laquelle R représente la chaîne hydrocarbonée de l'acide gras.

Pour le procédé, l'acide gras une fois activé peut être isolé ou non du milieu réactionnel avant d'effectuer la réaction de greffage.

Tous les acides gras sont susceptibles d'être activés par les moyens ci-dessus et susceptibles d'être employés dans le collagène greffé hydrophobe selon l'invention et son procédé de préparation.

Les acides gras sont biens connus de l'homme du métier. Les acides gras sont des acides carboxyliques aliphatiques comprenant une chaîne hydrocarbonée de longueur variable et un groupe carboxyle (-COOH). La chaîne hydrocarbonée comprend plus de 6 atomes de carbone, généralement entre 6 et 25 atomes de carbone, plus préférentiellement entre 10 et 22 atomes de carbone. Les acides gras peuvent être saturés ou insaturés, comprenant une ou plusieurs insaturations. Ils peuvent être linéaires ou ramifiés . Ils peuvent également être substitués par un ou plusieurs groupements fonctionnels notamment des groupes fonctionnels comprenant un ou plusieurs atomes d'oxygène, de souffre ou d'azote ou encore par un ou plusieurs atomes d'halogènes. Parmi les principaux acides gras linéaires on citera notamment les acides lauri que (C12), myristique (C14), palmitique (C16), stéarique (C18), oléique (C18, insaturé), et linoléique (C18, polyinsaturé) ou linolénique. L'acide laurique est le principal composant de l'huile de coco (45 - 50 %) d'huile de palme (45 - 55 %). Le beurre de nutmeg est riche en acide myristique qui constitue 60-75 % de sa teneur en acides gras. L'acide palmitique constitue entre 20 et 30 % de la plupart des graisses animales, mais aussi de graisses végétales. L'acide stéarique est le plus commun des acides gras naturels à longue chaîne, dérivé de graisses animales ou végétales. Enfin, l'acide oléique est le plus abondant des acides gras insaturés naturels.

Les acides gras ci-dessus peuvent être greffés seuls ou en mélanges sur le collagène selon l'invention.

De manière avantageuse, les acides gras sont choisis parmi les acides stéarique, palmitique et myristique et leurs mélanges en toutes proportions.

Des quantités variable et contrôlables d'acides gras sont introduites par réaction de l'acide gras activé sur les résidus lysines de la protéine. Une chaîne de collagène contient en théorie 30 résidus lysines. Il est donc possible de greffer de 0 à 30 molécules d'acides gras par chaîne alpha du collagène soit des taux de greffage de 0 à 100%.

Le greffage peut être effectué sur n'importe quel type de collagène et quelque soit sa structure : collagène natif, atelocollagène natif ou dénaturé ou de la gélatine. Cependant les taux de greffage maximum peuvent varier en fonction du contenu en lysine du collagène considéré et de l'accessibilité des résidus lysine au réactif, en particulier pour les collagènes non dénaturés. En fonction du niveau de structuration du collagène à greffer, différents solvants sont utilisés comme le méthanol, le dioxane, le DMSO ou un mélange de solvants en différentes proportions.

En cas de greffage sur du collagène non dénaturé, quel que soit le taux de greffage et l'acide gras greffé, le greffage a lieu de préférence sur du collagène en solution dans le méthanol ou en suspension dans le diméthylformamide (DMF). L'acide gras préalablement activé et avantageusement cristallisé comme explicité ci-dessus, est ajouté en solution au collagène dans un solvant approprié, par exemple un mélange DMF triéthylamine. Après réaction, le collagène greffé précipite et le précipité obtenu est lavé par un solvant approprié, en particulier par de l'acétone anhydre, et séché selon les méthodes usuelles, par exemple sous pression réduite.

En cas de greffage sur du collagène dénaturé, quel que soit le taux de greffage, et l'acide gras greffé, le collagène est séché une nuit sous pression réduite, dissout et dénaturé dans le diméthylsulfoxide (DMSO) à 70°C. En fonction du taux de greffage souhaité, l'acide gras activé est ajouté à la solution de collagène en conditions stoechiométriques en présence d'une base faible et préférentiellement la triéthylamine ou l'imidazole, dans le but de neutraliser environ 1,2 mEq d'H+ / g de collagène et de déprotoner les fonctions NH2 des résidus lysine. La solution est chauffée à 60°C jusqu'à dissolution des acides gras activés cristallisés. La réaction de greffage a lieu pendant 16 heures à température ambiante. Les solutions de collagènes greffés sont alors dialysées contre de l'eau acide pH 2-3 pour éliminer le DMSO et les bases. Le gel de collagène greffé obtenu est alors soit broyé dans 3 volumes d'acétone sèche puis séché sous pression réduite ; soit fondu à 60°C, séché à température ambiante et lavé à l'acétate d'éthyle pour éliminer les acides gras résiduels qui n'ont pas réagit.

Pour chaque collagène, le taux de greffage est calculé par la différence entre le taux d'amines libres dans le collagène de départ et le taux d'amines libres dans le collagène greffés. La méthode de dosage est dérivée des travaux de Kakade et al. [15]. La quantité d'amines libres est déterminée par la réaction de l'acide 2, 4, 6 trinitrobenzene sulfonique.

La solubilisation des collagènes greffés est effectuée dans l'eau ou dans un mélange eau / éthanol (à différentes proportions) ou dans l'acide acétique. Le solvant à utiliser dépend de la nature de l'acide gras et du taux de greffage. Lorsqu'on souhaite réticuler ce collagène en solution, l'agent réticulant est ajouté en solution aqueuse à la solution de collagène greffé.

Le collagène greffé selon l'invention peut être réticulé ou non, notamment pour la réalisation de matériaux aux propriétés antiadhésives vis-à-vis des cellules vivantes. Cette réticulation peut avoir lieu par les agents réticulant classiques (formaldéhyde, glutaraldéhyde,...) en particulier des réactifs mono, bi ou polyfonctionnels et particulièrement par les polysaccharides ramifiés oxydés (glycogène oxydé et/ou amylopectine oxydée par exemple).

Dans le cas des réticulations par les polysaccharides oxydés, la réticulation des collagènes greffés est obtenue par réaction des groupements aldéhydes du glycogène oxydé ou d'amylopectines oxydées avec les amines des résidus lysine restant après greffage sur le collagène. En modifiant le rapport CHO du polysaccharide / NH2 du collagène de 0,1 à 6, des taux de réticulation différents peuvent être obtenus. La réticulation a lieu par incubation du matériau obtenu par mélange du collagène greffé et du polysccharide oxydé à pH 9 puis réduction des groupements aldéhydes restants et des liaisons imines formés par un réducteur (borohydrure de sodium ou cyanoborohydrure de sodium par exemple).

Le procédé permet d'obtenir de manière aisée un matériau hydrophobe présentant des propriétés antiadhésives et aisément façonnable dans une forme appropriée selon l'usage qui en sera fait. En outre, le choix des acides gras employés et leur proportion permettront de moduler les propriétés antiadhésives de ce produit. Ainsi, il a été rapporté dans la littérature que l'activité inhibitrice des acides gras libres sur la croissance est plus marquée pour l'acide stéarique [18-20] que pour les acides myristiques et palmitiques [16, 17].

Après vérification de l'absence de toxicité indirecte des collagènes greffés, l'étude de leur activité sur l'adhésion et la croissance cellulaire a été réalisé sur la lignée continue fibroblastique MRC5. L'activité inhibitrice sur la croissance, découverte pour les acides gras seuls est retrouvée pour les collagènes greffés dans lesquels les acides gras ne sont pas libres. Cette activité est également plus marquée pour l'acide stéarique (85% d'inhibition) que pour les acides palmitiques et myristiques (65% d'inhibition). Cette action inhibitrice de la croissance cellulaire est exprimée dès que le taux de greffage atteint 1% et quel que soit l'acide gras. En ce qui concerne l'inhibition de l'adhésion, une activité inhibitrice est également observée dès un taux de greffage de 1% et quel que soit l'acide gras et elle atteint un maximum pour un taux variant de 20 à 30%.

La présente invention concerne également une composition pharmaceutique ou cosmétique comprenant du collagène hydrophobe greffé selon l'invention tel que défini ci-dessus et ci-après, en particulier une composition antiadhésive.

Le collagène greffé selon l'invention, quel que soit l'acide gras et le taux de greffage, peut être mis en forme pour donner des poudres, des solutions, des gels réticulés ou non, des éponges, des granulés, des films, des fils.

La présente concerne aussi un matériau antiadhésif comprenant du collagène hydrophobe greffé selon l'invention tel que défini ci-dessus et ci-après.

La composition de ces compositions, formes ou matériaux peut varier de 0,1 à 100% en collagène greffé. Des mélanges du collagène greffé avec d'autres biopolymères comme du collagène, de l'atelocollagène de la gélatine, des glycosaminoglycannes, des collagènes greffés avec le même acide gras mais à un taux de greffage différents, des collagènes greffés avec des acides gras différents, peuvent être effectués pour donner des produits présentant des propriétés physico-chimiques et biologiques variées.

En cas de confection d'un matériau réticulé ou non à partir des collagènes greffés, un plastifiant peut être ajouté jusqu'à 10% de la matière sèche. Le plastifiant est préférentiellement du glycérol mais d'autres produits tels que l'acide lactique peuvent être également utilisés.

Le collagène greffé selon l'invention, employé seul ou en mélange permettra notamment de :
- confectionner des matériaux composés entièrement ou en partie par un collagène modifié par greffage covalent d'acide gras ;
- confectionner des matériaux, éponges, gels, fils, granulés ou films transparents de collagène greffés réticulés par des agents de réticulation classique tels que le glutaraldéhyde et formaldéhyde ;
- confectionner des matériaux, éponges, gels, fils, granulés ou films transparents de collagène greffés réticulés par des polysaccharides oxydés ;
- confectionner des films d'une épaisseur allant de 20 à 200 µm ;
- confectionner des films bicouche réticulés dont une couche est composée de collagène quel que soit son niveau de structuration non modifié et réticulé et dont l'autre couche est composé de collagène greffé ou d'un mélange de collagène greffé et non greffé. Le collagène greffé pouvant également être réticulé ;
- confectionner des matériaux composites composés d'une face constituée d'une éponge de collagène greffé ou non et une face constituée d'un film de collagène greffé ou d'un mélange de collagène greffé et non greffé ;
- confectionner des matériaux biocompatible, non cytotoxiques réduisant l'adhésion cellulaire ;
- confectionner des matériaux composites constitués d'un treillis de polymères inertes (polyester, polyuréthane par exemple) imprégnés d'une couche poreuse ou non de collagène greffé ou d'un mélange de collagène greffé et non greffé.

Un tel collagène modifié par greffage d'acide gras peut être utilisé dans la confection de tout biomatériau où la réduction de l'adhésion cellulaire est souhaitée et tout particulièrement dans la confection de matériaux prévenant les adhérences post-chirurgicales, de prothèses vasculaires ou de lentilles intra-oculaires par exemple.

La présente invention concerne donc tout particulièrement l'utilisation des collagènes greffés ou d'un mélange de collagène greffé et non greffé pour la mise en forme d'un matériau prévenant les adhérences post-opératoires. On pourra ainsi utiliser le collagène greffé selon l'invention, seul ou en mélange avec d'autres collagènes et notamment des collagènes greffés pour la fabrication de films mon ou bicouches. Elle concerne aussi l'association de collagène greffé ou d'un mélange de collagène greffé et non greffé avec des matériaux existants tels que des treillis de polymère par exemple pour le renfort de paroi abdominale. On pourra utiliser le collagène selon l'invention, seul ou en mélange, pour l'imprégnation de tels matériaux.

La présente invention concerne aussi les films mono ou bicouches ainsi obtenus pour l'imprégnation des treillis avec le collagène selon l'invention.

La présente invention concerne donc aussi une prothèse chirurgicale, notamment prothèse vasculaire, comprenant un matériau antiadhésif tel que défini ci-dessus et ci-après. Elle concerne également une lentille intraoculaire comprenant ledit matériau antiadhésif.

Ce document concerne enfin l'utilisation du collagène hydrophobe tel que défini ci-dessus et ci-dessous en thérapie.

Les exemples de réalisation ci-dessous permettent d'illustrer l'invention. A moins qu'il ne soit entendu autrement, les informations relatives à la réalisation des exemples ci-après, en particulier les informations concernant la mise en ouvre des procédés de préparation des collagènes greffés selon l'invention peuvent s'étendre à l'ensemble des collagènes greffés définis ci-dessus.

Le collagène dénaturé mis en réaction pour le greffage est extrait selon un procédé précédemment décrit [21] et extrait de préférence d'un tissu porcin. Pendant la purification, le collagène peut être traité avec une solution d'hydroxyde de sodium 1M à 20°C pendant 1h sans modification détectable de sa structure chimique et de ses propriétés biologiques. Ce traitement est recommandé pour détruire les agents pathogènes conventionnels et non conventionnels [22].

L'agent réticulant et plus particulièrement le glycogène oxydé ou l'amylopectine oxydé est obtenu par oxydation périodique du polysaccharide en milieu aqueux selon Abdel-Akher et al [23] modifié par Rousseau et al [21]. La détermination du degré d'oxydation est réalisée en s'inspirant de la méthode Zhao et al [24].

### Exemple 1 : préparation du stearoyl imidazolide cristallisé

Pour la préparation d'environ 2,4 g de stearoyl imidazolide, 2g d'acide stéarique sont dissout dans 12 ml de dimétylformamide anhydre à chaud (40°C). La réaction étant stoechiométrique, on prévoit d'ajouter la quantité de carbonyldiimidazole correspondante avec 5% d'excès, ici 1,34 g. Pratiquement, la première moitié de la quantité de CDI est ajoutée dans la solution. Les cristaux de stearoyl imidazolide précipitent. Ils sont solubles à chaud (40°C). Après redissolution, le reste de CDI est ajouté. Après 2 heures à température ambiante, la précipitation des cristaux de stéaroyl imidazolide est obtenue en maintenant le milieu réactionnel à 0°C pendant 3 heures. Le précipité est récolté par filtration, puis lavé par 24 ml de DMF froid et 12 ml d'éthanol et séché. La molécule obtenue présente un poids moléculaire de 334,5 g/mol. Sa formule chimique est la suivante :

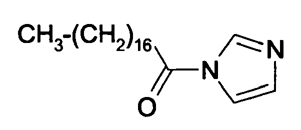

### Exemple 2 : activation de l'acide gras par le N-hydroxysuccinimide

Dissolution de l'acide gras à 10-20% dans du dioxane à 20°C puis dissolution de N-hydroxysuccinimide (1,3 eq/eq d'acide gras) et addition du cyclohexylcarbodiimide (0,98 eq/ eq d'acide gras). Après 2 à 3 heures de réaction à 20°C, l'urée formée par la réaction est éliminé par filtration et le filtrat est évaporé pour donner un ester activé qui est recristallisé dans le DMF.

### Exemple 3 : greffage de l'acide gras activé sur le collagène dénaturé : exemple du greffage du myristoylimidazolide cristallin sur l'atelocollagène dénaturé, taux de greffage théorique 20%

5 g d'atelocollagène anhydre contenant 1,6 mmol de résidus lysine sont mis en dissolution dans 50ml de DMSO anhydre à 60°C. 0,322 mmoles (99mg) de myristoylimidazolide (PM 306,5 g/mol) correspondant à 20% des lysines du collagène mis en réaction sont ajoutées à la solution de collagène et le mélange est chauffé à 60°C jusqu'à dissolution des cristaux d'acide gras activé. 6 mmoles de triéthylamine sont ajoutées afin de déprotoner les fonctions epsilon amines des lysines et le milieu est agité à 20°C pendant 16 heures. A l'issue de la réaction, le milieu réactionnel est dialysé contre de l'eau jusqu'à élimination totale de la triéthylamine et DMSO. Le gel formé au cours de la dialyse est fondu à 60°C et la solution obtenue est déshydratée sous flux d'air sec pour donner des films. Ceux ci peuvent être lavé par de l'acétate d'éthyle pour extraire les acides gras activés ou non qui n'auraient pas réagit. Le rendement est compris entre 90 et 99%. Le taux de greffage est déterminé par dosage des amines epsilon lysines restantes.

Les gels formés au cours de la dialyse peut être également broyé dans 3 volumes d'acétone sèche : le collagène greffé est alors obtenu sous forme de poudre.

### Exemple 4 : greffage de l'acide gras activé sur le collagène dénaturé : exemple du greffage du palmitoylimidazolide sans isolement de l'imidazolide sur l'atelocollagène dénaturé, taux de greffage théorique 40%

10 g d'atelocollagène contenant 3,2 mmol de résidus lysines sont mis en dissolution dans 100 ml de DMSO anhydre à 60°C. 450 mg d'acide palmitique sont dissout dans du DMSO anhydre à 1,7% à chaud 60°C. 1,4 mmoles de CDI sont ajoutés à la solution d'acide gras. La réaction d'activation a lieu pendant 2 heures. Les cristaux de palmitoylimidazolide (320,5 g/mol) sont solubilisés à 60°C et le volume correspondant à 1,28 mmol de palmitoylimidazolide est ajouté à la solution de collagène. La solution est chauffée à 60°C jusqu'à dissolution des cristaux d'acide gras activés. 12 mmoles de triéthylamine sont ajoutées afin de déprotoner les fonctions epsilon amines des lysines et le milieu est agité à 20°C pendant 16 heures. A l'issue de la réaction, le milieu réactionnel est dialysé contre de l'eau jusqu'à élimination totale de la triéthylamine et DMSO. Le gel formé au cours de la dialyse est fondu à 60°C et la solution obtenue est déshydratée sous flux d'air sec pour donner des films. Ceux-ci peuvent être lavé par de l'acétate d'éthyle pour extraire les acides gras activés ou non qui n'auraient pas réagit. Le rendement est compris entre 90 et 99%. Le taux de greffage est déterminé par dosage des amines epsilon lysines restantes.

Les gels formés au cours de la dialyse peut être également broyé dans 3 volumes d'acétone sèche : le collagène greffé est alors sous forme de poudre.

### Exemple 5: solubilisation des collagènes greffés avec des taux variables d'acides stéarique, myristique et palmitique

Tous les collagènes dénaturés greffés avec l'acide stéarique, palmitique et myristique (à l'exception des taux de greffage supérieur à 98%) sont solubles à chaud (60°C) dans un mélange eau / éthanol (75 : 25). Pour la préparation de solutions de 1 à 2%, le collagène est dissout dans 50% du volume final dans un mélange eau éthanol (50 : 50). Le mélange est chauffé à 60°C. Une fois le collagène dissout, le milieu est dilué au ½ par de l'eau.

Pour les collagènes dénaturés greffés à un taux de 98% d'acides gras, la dissolution n'est possible que dans de l'acide acétique pur.

Par contre, pour des greffages inférieurs ou égaux à 30%, les collagènes dénaturés sont hydrosolubles.

### Exemple 6 : préparation de matériaux à base d'atelocollagène dénaturé greffé par un acide gras. Exemple de réticulation de l'atelocollagène par le glycogène oxydé

### a) Exemple de confection d'un film dans des plaques de culture pour tests d'adhésion :

Une solution est préparée en mélangeant des solutions de collagène greffé en concentration allant de 1 à 2% dans un mélange eau / éthanol (25 / 75) avec du glycogène oxydé à 0,8 moles de CHO / mole de saccharide pour obtenir un ratio de 2 CHO du glycogène oxydé / 1 NH2 du collagène [21]. La solution de collagène est obtenue par chauffage à 60°C jusqu'à dissolution du collagène greffé. Après refroidissement, la solution de glycogène oxydé est ajoutée puis le glycérol à raison de 10% par rapport à la matière sèche. 1,5 ml de la solution finale obtenue sont coulés au fond des puits de plaque de culture 6 puits. La solution est évaporée sous flux d'air contrôlé selon les procédés habituels bien connus de réalisation des films. La réticulation est obtenue par immersion des films dans un bain de tampon carbonate de sodium 0,1M pH9. Les films sont lavés à l'eau distillée, immergés dans une solution réductrice de borohydrure de sodium à 400 mg/L, lavés à l'eau distillée, plongés dans du PBS puis séchés sous flux d'air contrôlé.

### b) Exemple de confection de films de collagène greffé à 20% par de l'acide stéarique et réticulé par du glycogène oxydé selon le ratio 0,4 moles CHO /mole de NH2:

Pour l'obtention de films de 12 cm par 12 cm d'une épaisseur de 45µm, une solution aqueuse à 1,75% de collagène greffé à 20% par de l'acide stéarique est réalisée. Après refroidissement, le glycogène oxydé est ajouté à raison de 0,4 CHO / NH2 en solution. Le glycérol est ensuite ajouté. La solution finale est coulée sur des boites de polystyrène de 144 cm². La solution, après gélification est évaporée sous flux d'air contrôlé. Une fois secs, les films sont immergés dans du tampon carbonate 0,1M pH 9 pendant 45minutes, lavés avec de l'eau distillée, réduit par une solution de borohydrure de sodium à 400 mg/L, lavés à l'eau distillée, plongés dans du PBS puis séchés sous flux d'air contrôlé. Ces films peuvent être stérilisés par rayonnement béta ou gamma.

### Exemple 7 : exemple de confection de films de collagène greffé à 30% par de l'acide stéarique et réticulé par du glutaraldéhyde

Pour l'obtention de films de 12 cm par 12 cm d'une épaisseur de 45µm, une solution aqueuse à 1,75% de collagène greffé à 30% par de l'acide stéarique est réalisée. Le glycérol est ajouté à raison de 10% de la matière sèche de collagène. La solution finale est coulée sur des boites de polystyrène de 144 cm². La solution, après gélification est évaporée sous flux d'air contrôlé. Le film sec est alors immergé dans une solution de glutaraldéhyde à 0,5% pendant 18 heures à pH 7, puis une solution de Tris, rincé en PBS puis séché. Ce film peut être stérilisé par rayonnement béta ou gamma.

### Exemple 8 : fabrication d'une éponge lyophilisé de collagène greffée avec de l'acide palmitique à 13%

Une solution aqueuse de collagène greffé par 8% d'acides palmitiques en concentration de 1 à 2% dans l'eau est obtenue par chauffage à 60°C pendant 1 heure. La solution est ensuite coulée dans une barquette métallique et congelée à -70°C. Après 48 heures de lyophilisation, des éponges sont obtenues. La porosité moyenne dépend de la concentration en collagène et de la température de congélation.

### Exemple 9 : application du collagène greffé à la confection de biomatériaux implantables

### a) étude in vitro de cytotoxicité :

Des échantillons de films de collagènes greffés par des acides gras tels que l'acide stéariques, palmitiques et myristique réticulés sont testés en ce qui concerne leur cytotoxicité envers des fibroblastes.
Aucune cytotoxicité indirecte n'est observée quelque soit le taux de greffage des différents acides gras.

### b) étude in vitro de croissance cellulaire :

Des échantillons de films de collagènes greffés par des acides gras tels que l'acide stéariques, palmitiques et myristique réticulés sont testés en ce qui concerne la croissance des fibroblastes à leur contact. Après 5 jours de croissance cellulaire, au contact des films, les cellules sont détachées par de la trypsine et la viabilité cellulaire est mesurée par réaction avec le MTT.

Pour les films réalisés avec des collagènes greffés avec de l'acide palmitique et myristique, quel que soit le taux de greffage, la croissance cellulaire est diminuée d'en moyenne environ 65%.

Pour les films réalisés avec des collagène greffés avec de l'acide stéarique, quel que soit le taux de greffage, la croissance cellulaire est diminuée d'en moyenne environ 85%.

### c) étude in vitro d'adhésion cellulaire :

Des échantillons de films de collagènes greffés par des acides gras tels que l'acide stéariques, palmitiques et myristique réticulés sont testés en ce qui concerne l'adhésion des fibroblastes à leur contact. Des cinétiques de détachement à la trypsine ont été réalisées. Dans chaque extrait, la viabilité cellulaire est mesurée par réaction avec le MTT.

Quel que soit le taux de greffage et l'acide gras greffé, l'adhésion cellulaire est diminuée. Un maximum de diminution de l'adhésion cellulaire est observé autour de 25 à 30% de taux de greffage.

### d) étude in vivo de biodégradation :

Des échantillons de films de collagènes greffés par des acides gras tels que l'acide stéariques, palmitiques et myristique réticulés sont implantés en position sous cutanée chez la souris.

La biodégradation des matériaux en fonction du taux de réticulation est étudiée. Des études histologiques permettent de caractériser la réaction de l'hôte.

Quel que soit le taux de réticulation et de greffage, aucune réaction pathologique n'est observée. La mobilisation des cellules du système immunitaire est normale. Aucune coque fibreuse n'est observée autour de l'implant.

### e) étude in vivo d'immunogénicité :

Un protocole d'immunisation de lapins avec un broyat de collagène greffé à 26% avec de l'acide stéarique a été réalisé.

Après 90 jours d'immunisation, aucune production d'anticorps dirigés contre le collagène greffé n'a été mise en évidence.

### Exemple 10 : greffage de l'acide stéarique sur de l'atelocollagène non dénaturé.

### a) dans le méthanol

A une solution homogène de 500 mg d'atélocollagène (0,16 mmol. de lysine) dans 30 ml de méthanol anhydre est ajoutée une solution de stéaroylimmidazole (100 mg, 0,3 mmol.) dans 5 ml de dioxane contenant 150 µl de triéthylamine (1,1 mmol.). Le gel obtenu est dispersé finement et la suspension est agitée 24h à 20°. Le précipité est essoré et lavé par de l'acétone puis séché sous pression réduite.

### b) dans le DMF

A une suspension de 1 g (0,32 mmol. de lysine) de collagène en poudre fine dans 20 ml de DMF sont ajoutés 20 ml de dioxane contenant 200 mg (0,6 mmol.) de stéaroylimmidazole et 300 µl de triéthylamine (2,2 mmol.). Après 48h de réaction à 30°, le collagène est récolté par filtration, lavé par de l'acétone anhydre et séché sous pression réduite.

### Références :

1. Myles, J.L., B.T. Burgess, and R.B. Dickinson, Modification of the adhesive properties of collagen by covalent grafting with RGD peptides. J Biomater Sci Polym Ed, 2000. 11(1): p. 69-86.
2. Park, J.C., et al., Type I atelocollagen grafting onto ozone-treated polyurethane films: cell attachment, proliferation, and collagen synthesis. J Biomed Mater Res, 2000. 52(4): p. 669-77.
3. Lee, J.H., et al., Interaction of cells on chargeable functional group gradient surfaces. Biomaterials, 1997. 18(4): p. 351-8.
4. Sugimoto, Y., Effect on the adhesion and locomotion of mouse fibroblasts by their interacting with differently charged substrates. A quantitative study by ultrastructural method. Exp Cell Res, 1981. 135(1): p. 39-45.
5. Marois, Y., M.F. Sigot-Luizard, and R. Guidoin, Endothelial cell behavior on vascular prosthetic grafts: effect of polymer chemistry, surface structure, and surface treatment. Asaio J, 1999. 45(4): p. 272-80.
6. Lampin, M., et al., Correlation between substratum roughness and wettability, cell adhesion, and cell migration. JBiomed Mater Res, 1997. 36(1): p. 99-108.
7. Kleinman, H.K., R.J. Klebe, and G.R. Martin, Role of collagenous matrices in the adhesion and growth of cells. J Cell Biol, 1981. 88(3): p. 473-85.
8. Massia, S.P. and J.A. Hubbell, Human endothelial cell interactions with surface-coupled adhesion peptides on a nonadhesive glass substrate and two polymeric biomaterials. J Biomed Mater Res, 1991. 25(2): p. 223-42.
9. Miyata, T., et al., A biodegradable antiadhesion collagen membrane with slow release heparin. ASAIO Trans, 1988. 34(3): p. 687-91.
10. Duncan, G., et al., Thapsigargin-coated intraocular lenses inhibit human lens cell growth. Nat Med, 1997. 3(9): p. 1026-8.
11. Tziampazis, E., J. Kohn, and P.V. Moghe, PEG-variant biomaterials as selectively adhesive protein templates: model surfaces for controlled cell adhesion and migration. Biomaterials, 2000.21(5): p. 511-20.
12. DeFife, K.M., et al., Effects of photochemically immobilized polymer coatings on protein adsorption, cell adhesion, and the foreign body reaction to silicone rubber. J Biomed Mater Res, 1999. 44(3): p. 298-307.
13. Werner, L., et al., Evaluation of teflon-coated intraocular lenses in an organ culture method JBiomed Mater Res, 1999. 46(3): p. 347-54.
14. Altankov, G. and T. Groth, Fibronectin matrix formation by human fibroblasts on surfaces varying in wettability. J Biomater Sci Polym Ed, 1996. 8(4): p. 299-310.
15. Kakade, M.L. and I.E. Liener, Determination of available lysine in proteins. Anal Biochem, 1969. 27(2): p. 273-80.
16. Legrand, P. and V. Rioux, De l'acide myristique à la myristoylation des protéines. CERIN Cholé-doc, 2000. N° 59.
17. Boutin, J.A., Myristoylation. Cell Signal, 1997. 9(1): p. 15-35.
18. Wickramasinghe, N.S., et al., Stearate inhibition of breast cancer cell proliferation. A mechanism involving epidermal growth factor receptor and G-proteins. Am J Pathol, 1996. 148(3): p. 987-95.
19. Johanning, G.L. and T.Y. Lin, Unsaturated fatty acid effects on human breast cancer cell adhesion. Nutr Cancer, 1995. 24(1): p. 57-66.
20. Singh, R.K., et al., Stearate inhibits human tumor cell invasion. Invasion Metastasis, 1995. 15(3-4): p. 144-55.
21. Rousseau, C.F. and C.H. Gagnieu, In vitro cytocompatibility of porcine type I atelocollagen crosslinked by oxidized glycogen. Biomaterials, 2002. 23(6): p. 1503-10.
22. Dormont, D., [Nature and physicochemical and biological properties of non conventional transmissible agents or prions: consequences for public health]. Pathol Biol (Paris), 1995. 43(2): p. 124-36.
23. Abdel-Akher, M. and F. Smith, Reduction of the products of periodate oxidation of carbohydrates. VII. the constitution of glycogen. Arch Biochem Biophys, 1958. 78(2): p. 451-9.
24. Zhao, H. and N.D. Heindel, Détermination of degree of substitution of formyl groups in polyaldehyde dextran by the hydroxylamine hydrochloride method Pharm Res, 1991. 8(3): p. 400-2.

## Revendications

1. Collagène hydrophobe greffé comprenant des acides gras greffés sur le collagène par liaison covalente, pour son utilisation en thérapie.

2. Collagène hydrophobe greffé selon la revendication 1, **caractérisé en ce que** les acides gras sont greffés sur les résidus amines libres de la chaîne alpha du collagène.

3. Collagène hydrophobe greffé selon l'une des revendications 1 ou 2, **caractérisé en ce que** le pourcentage d'acides gras par rapport aux résidus aminés libres de la chaîne alpha du collagène est compris entre 1 et 100%, de préférence supérieur à environ 10% et inférieur à environ 85%.

4. Collagène hydrophobe greffé selon la revendication 3, **caractérisé en ce que** le pourcentage d'acides gras est compris entre 15 et 50%, plus préférentiellement compris entre 20 et 30%.

5. Collagène hydrophobe greffé selon l'une des revendications 1 à 4, **caractérisé en ce que** les acides gras sont choisis parmi les acides stéarique, palmitique et myristique et leurs mélanges en toutes proportions.

6. Collagène hydrophobe greffé selon l'une des revendications 1 à 5, **caractérisé en ce que** le collagène est choisi parmi le collagène natif, l'atelocollagène natif, le collagène ou l'atelocollagène dénaturé ou de la gélatine.

7. Collagène hydrophobe greffé selon l'une des revendications 1 à 6, **caractérise en ce que** le collagène greffé est réticulé.

8. Collagène hydrophobe greffé selon la revendication 7, **caractérisé en ce que** le collagène greffé est réticulé par des polysaccharides ramifiés oxydés, notamment choisis parmi le glycogène oxydé et les amylopectines oxydées.

9. Collagène hydrophobe greffé selon l'une des revendications 1 à 8, pour son utilisation dans la réduction de l'adhésion cellulaire.

10. Collagène hydrophobe greffé selon l'une des revendications 1 à 8, pour son utilisation dans la prévention des adhérences post-opératoires.

11. Utilisation d'un collagène hydrophobe greffé selon l'une des revendications 1 à 8 pour conférer une propriété antiadhésive à un matériau, ledit matériau comprenant le collagène hydrophobe greffé seul ou en mélange.

12. Prothèse chirurgicale, notamment prothèse vasculaire, comprenant un matériau antiadhésif, ledit matériau antiadhésif comprenant du collagène hydrophobe greffé selon l'une des revendications 1 à 8.

13. Lentille intraoculaire comprenant un matériau antiadhésif, ledit matériau antiadhésif comprenant du collagène hydrophobe greffé selon l'une des revendications 1à 8.

14. Film mono ou bicouche comprenant un matériau antiadhésif, ledit matériau antiadhésif comprenant du collagène hydrophobe greffé selon l'une des revendications 1à 8.

15. Treillis pour le renfort des parois abdominales **caractérisé en ce qu'**il est imprégné par un matériau antiadhésif, ledit matériau antiadhésif comprenant du collagène hydrophobe greffé selon l'une des revendications 1à 8.

16. Composition pharmaceutique **caractérisée en ce qu'**elle comprend du collagène hydrophobe greffé selon l'une des revendications 1 à 8.

## Claims

1. A grafted hydrophobic collagen comprising fatty acids grafted onto the collagen by covalent bonding, for its use in therapy.

2. The grafted hydrophobic collagen according to claim 1, **characterized in that** the fatty acids are grafted onto the free amine residues of the collagen alpha chain.

3. The grafted hydrophobic collagen according to any of claims 1 or 2, **characterized in that** the percentage of fatty acids relative to the free amine residues of the collagen alpha chain is comprised between 1 and 100%, preferably greater than about 10% and less than about 85%.

4. The grafted hydrophobic collagen according to claim 3, **characterized in that** the percentage of fatty acids is comprised between 15 and 50%, more preferably between 20 and 30%.

5. The grafted hydrophobic collagen according to any of claims 1 to 4, **characterized in that** the fatty acids are selected from among stearic, palmitic and myristic acids and their mixtures in any proportions.

6. The grafted hydrophobic collagen according to any of claims 1 to 5, **characterized in that** the collagen is selected from among native collagen, native atelocollagen, denaturated collagen or atelocollagen, or gelatin.

7. The grafted hydrophobic collagen according to any of claims 1 to 6, **characterized in that** the grafted collagen is crosslinked.

8. The grafted hydrophobic collagen according to claim 7, **characterized in that** the grafted collagen is crosslinked with branched, oxidized polysaccharides, in particular selected from among oxidized glycogen and oxidized amylopectins.

9. The grafted hydrophobic collagen according to any of claims 1 to 8, for its use in reduction of cell adhesion.

10. The grafted hydrophobic collagen according to any of claims 1 to 8, for its use in the prevention of post-operative adherences.

11. The use of a grafted hydrophobic collagen according to any of claims 1 to 8, for imparting an anti-adhesive property to a material, said material comprising the grafted hydrophobic collagen alone or as a mixture.

12. A surgical prosthesis, notably a vascular prosthesis, comprising an anti-adhesive material, said anti-adhesive material comprising grafted hydrophobic collagen according to any of claims 1 to 8.

13. An intraocular lens comprising an anti-adhesive material, said anti-adhesive material comprising grafted hydrophobic collagen according to any of claims 1 to 8.

14. A single-layer or bi-layer film comprising an anti-adhesive material, said anti-adhesive material comprising grafted hydrophobic collagen according to any of claims 1 to 8.

15. A lattice for the reinforcement of abdominal walls, **characterized in that** it is impregnated with an anti-adhesive material, said anti-adhesive material comprising grafted hydrophobic collagen according to any of claims 1 to 8.

16. A pharmaceutical composition **characterized in that** it comprises grafted hydrophobic collagen according to any of claims 1 to 8.

## Patentansprüche

1. Gegraftetes hydrophobes Kollagen, umfassend Fettsäuren, gegraftet auf das Kollagen durch kovalente Bindung, zur Verwendung in der Therapie.

2. Gegraftetes hydrophobes Kollagen nach Anspruch 1, **dadurch gekennzeichnet, dass** die Fettsäuren auf die freien Aminoreste der Alphakette des Kollagens gegraftet sind.

3. Gegraftetes hydrophobes Kollagen nach einem der Ansprüche 1 oder 2, **dadurch gekennzeichnet, dass** der Prozentsatz an Fettsäuren bezüglich der freien Aminoreste der Alphakette des Kollagens zwischen 1 und 100 %, vorzugsweise über ungefähr 10 % und unter ungefähr 85 % liegt.

4. Gegraftetes hydrophobes Kollagen nach Anspruch 3, **dadurch gekennzeichnet, dass** der Prozentsatz an Fettsäuren zwischen 15 und 50 %, insbesondere zwischen 20 und 30 % liegt.

5. Gegraftetes hydrophobes Kollagen nach einem der Ansprüche 1 bis 4, **dadurch gekennzeichnet, dass** die Fettsäuren unter der Stearinsäure, Palmitinsäure und Myristinsäure und ihren Mischungen in allen Proportionen ausgewählt werden.

6. Gegraftetes hydrophobes Kollagen nach einem der Ansprüche 1 bis 5, **dadurch gekennzeichnet, dass** das Kollagen unter dem nativen Kollagen, dem nativen Atelokollagen, dem denaturierten Kollagen oder Atelokollagen oder der Gelatine ausgewählt wird.

7. Gegraftetes hydrophobes Kollagen nach einem der Ansprüche 1 bis 6, **dadurch gekennzeichnet, dass** das gegraftete Kollagen vernetzt ist.

8. Gegraftetes hydrophobes Kollagen nach Anspruch 7, **dadurch gekennzeichnet, dass** das gegraftete Kollagen von oxidierten verzweigten Polysacchariden vernetzt ist, insbesondere ausgewählt unter dem oxidierten Glykogen und den oxidierten Amylopectinen

9. Gegraftetes hydrophobes Kollagen nach einem der Ansprüche 1 bis 8 für die Verwendung bei der Verringerung der Zelladhäsion.

10. Gegraftetes hydrophobes Kollagen nach einem der Ansprüche 1 bis 8 für die Verwendung bei der Prävention der postoperativen Verwachsungen.

11. Verwendung eines gegrafteten hydrophoben Kollagens nach einem der Ansprüche 1 bis 8, um einem Material eine antihaftende Eigenschaft zu verleihen, wobei das Material das gegraftete hydrophobe Kollagen allein oder in einer Mischung umfasst.

12. Chirurgische Prothese, insbesondere Gefäßprothese, umfassend ein antihaftendes Material, wobei das antihaftende Material ein gegraftetes hydrophobes Kollagen nach einem der Ansprüche 1 bis 8 umfasst.

13. Intraokulare Linse, umfassend ein antihaftendes Material, wobei das antihaftende Material ein gegraftetes hydrophobes Kollagen nach einem der Ansprüche 1 bis 8 umfasst.

14. Ein- oder doppelschichtiger Film, umfassend ein antihaftendes Material, wobei das antihaftende Material ein gegraftetes hydrophobes Kollagen nach einem der Ansprüche 1 bis 8 umfasst.

15. Gitter für die Verstärkung der Bauchdecken, **dadurch gekennzeichnet, dass** es mit einem antihaftenden Material imprägniert ist, wobei das antihaftende Material ein gegraftetes hydrophobes Kollagen nach einem der Ansprüche 1 bis 8 umfasst.

16. Pharmazeutische Zusammensetzung, **dadurch gekennzeichnet, dass** sie ein gegraftetes hydrophobes Kollagen nach einem der Ansprüche 1 bis 8 umfasst.
